# FASCICULE DE BREVET EUROPEEN

(11) **EP 0 897 907 B1**
(45) Date de publication et mention de la délivrance du brevet: **05.12.2001**
(21) Numéro de dépôt: 98402034.7
(22) Date de dépôt: 11.08.1998
(51) Int. Cl.: C07C 201/16

(54) **Procédé de récupération d'acide nitrique compris dans un mélange de composés aromatiques dinitrés**
Verfahren zur Rückgewinnung von Salpetersaüre in eine Mischung von aromatischen Dinitroverbindungen
Process for the recovery of nitric acid in a mixture of aromatic dinitro compounds

(30) Priorité: 13.08.1997 FR 9710347
(43) Date de publication de la demande: 24.02.1999
(73) Titulaire: RHODIA CHIMIE, 92408 Courbevoie Cédex (FR)
(72) Inventeur: Marion, Philippe, 69390 Vernaison (FR); Metivier, Pascal, 69110 Sainte Roy Le Lyon (FR)
(74) Mandataire: Wattremez, Catherine

(56) Documents cités:
- EP-A- 0 279 312
- EP-A- 0 736 514
- DE-A- 2 338 479
- DE-C- 4 216 416

## Description

Le procédé selon l'invention concerne la récupération d'acide nitrique contenu dans un flux comprenant des composés aromatiques dinitrés.

Plus particulièrement, la présente invention a pour objet la récupération d'acide nitrique contenu dans un flux organique comprenant majoritairement des composés aromatiques dinitrés.

Les procédés de préparation de composés dinitrés aromatiques sont exploités industriellement depuis de nombreuses années. Ces composés sont en effet des intermédiaires à la préparation de diamines aromatiques, elles-mêmes utilisées pour la synthèse des isocyanates correspondants. Les isocyanates sont employés pour la synthèse de polyuréthannes, dont les applications sont très nombreuses.

Résumées rapidement, les réactions de dinitration sont mises en oeuvre, la plupart du temps, en deux étapes, la première consistant à préparer les composés mononitrés, la seconde, les composés dinitrés. Outre le composé aromatique à faire réagir, on emploie un acide nitrant, qui est en général un mélange acide nitrique / acide sulfurique, l'acide sulfurique étant un catalyseur de la réaction.

A l'issue de chaque étape de nitration, le composé dinitré est séparé de l'acide résiduaire. Cette opération a lieu classiquement par décantation directe ou par centrifugation du mélange réactionnel.

A l'exception des composés mononitrés destinés à la synthèse de composés dinitrés, les composés dinitrés obtenus après séparation d'avec l'acide nitrant (aussi appelés composés dinitrés bruts) ne peuvent être utilisés tels quels, car ils contiennent toujours, à l'état dissous, une fraction d'acide nitrant, de même que des impuretés organiques.

Ces problèmes liés à la purification des composés dinitrés aromatiques, et plus particulièrement à la séparation et à la récupération de l'acide dissous, ont fait l'objet de nombreuses études.

En effet, récupérer ces acides résiduaires représente un enjeu économique non négligeable, étant donnés les tonnages de composés dinitrés. Par ailleurs, leur récupération a aussi un impact sur l'environnement car elle a notamment pour conséquence de limiter les rejets aqueux. De plus, la récupération des acides permet de diminuer les coûts nécessaires pour traiter les eaux résiduaires que l'on ne peut rejeter telles quelles car elles sont polluées avec des sels, tels que les sulfates et surtout les nitrates.

Les méthodes connues de séparation et de récupération de l'acide nitrique dissous ont toutes trait à l'augmentation de l'efficacité de lavage des composés dinitrés.

Selon une première possibilité, décrite notamment dans le brevet européen EP 279 312, on effectue le lavage du dinitrotoluène brut avec une quantité d'eau très faible. De cette façon, les eaux de lavages, chargées en acides nitrique et sulfurique, sont suffisamment concentrées pour pouvoir être renvoyées directement dans le processus de nitration. Cependant, un tel procédé présente l'inconvénient de nécessiter l'emploi d'un appareillage particulier pour séparer la phase aqueuse de la phase organique, en l'occurrence, un coalesceur. En effet, du fait de la très faible teneur en eau utilisée pour le lavage, la décantation est difficile. En outre, les rendements d'extraction sont au plus de 72 %.

Une autre possibilité, ayant fait l'objet de la demande de brevet européen EP 736 514, consiste à laver en plusieurs étapes à contre-courant, le dinitrotoluène brut avec de l'eau chargée en acides employés pour la réaction de nitration. Les eaux récupérées peuvent être recyclées dans le procédé de nitration, sans concentration préalable, ou de préférence avec une concentration préalable. Ce procédé ne présente cependant, pas toutes les garanties de sécurité souhaitées. En effet, l'étape de concentration des eaux acides, qui s'avère dans la majeure partie des cas, nécessaire, présente quelques risques. En effet, la phase aqueuse distillée comprend à la fois l'acide nitrant et du dinitrotoluène dissous. Par conséquent, on se trouve dans des conditions de nitration d'un dinitrotoluène, qui conduit au trinitrotoluène dont on connaît les propriétés particulières. De plus, le dinitrotoluène et ses isomères présents dans la phase aqueuse acide issue du lavage, ne sont pas récupérés, dans les cas où la concentration desdites eaux est mise en oeuvre. En effet, ces composés sont entraînés avec l'eau lors de la distillation où ils sont perdus. Par ailleurs, les composés dinitrés peuvent être la cause d'un bouchage ou d'un encrassement de la colonne de concentration car ils sont condensés en tête de colonne et se retrouvent alors sous une forme solide. Ils peuvent de même se retrouver dans les eaux destinées à être rejetées, causant alors une pollution ou un surcoût car il est nécessaire de les éliminer. Enfin, pour avoir de bons rendements en récupération d'acides nitrique et sulfurique, il est nécessaire de mettre en oeuvre au moins 2 ou 3 étages de lavages, en d'autres termes au moins 2 ou 3 unités de mélangeur-décanteur comprenant pour chacune des unités un recyclage de la phase aqueuse. Par conséquent, le procédé est de mise en oeuvre complexe et nécessite un investissement important.

La présente invention a pour objet de proposer une alternative simple aux procédés connus de récupération de l'acide nitrique.

Ainsi le procédé selon rinvention consiste à traiter un flux obtenu par (1) réaction de dinitration de composés aromatiques avec un acide nitrant comprenant au moins de l'acide nitrique, puis (2) séparation de l'acide nitrant ; ce flux comprenant un composé dinitré aromatique dans lequel reste dissous une fraction d'acide nitrant. Le procédé selon l'invention comprend la mise en oeuvre des étapes suivantes :
(a) On effectue une distillation ou un stripping du flux précité,
(b) On recycle l'acide nitrique récupéré en tête de l'étape (a), dans le procédé de nitration,
(c) On traite le flux débarrassé de l'acide nitrique et comprenant entre autres les composés aromatiques dinitrés par lavage.

Il a été trouvé de façon totalement inattendue que le fait de distiller les composés aromatiques dinitrés n'entraînait pas de dégradation de ces derniers, par exemple par réaction desdits composés nitrés avec l'acide nitrant présent dans le flux, ce qui aurait pu entraîner la formation de produits dangereux et donc être la cause de difficultés de mise en oeuvre du procédé, sur le plan de la sécurité.

Il est à noter à ce titre, qu'aucun procédé décrit dans l'art antérieur ne suggère d'effectuer un tel traitement de distillation directement sur un flux comprenant majoritairement des composés aromatiques dinitrés. En effet, les distillations de ce genre sont réalisées classiquement sur des effluents aqueux comprenant seulement des quantités minimes de produits organiques nitrés, comme cela apparaît notamment avec la demande de brevet européen EP 736 514.

Tout au contraire, le procédé selon l'invention est bien plus satisfaisant sur le plan de la sécurité. En effet, parce que le temps de séjour dans l'appareillage où est mis en oeuvre la distillation est faible, l'encours en produits aromatiques nitrés dans l'installation est considérablement diminué, ce qui représente un avantage très important. Or cette caractéristique n'est pas atteinte dans le cas des procédés classiques de lavages des composés aromatiques nitrés bruts.

En outre, le procédé selon l'invention permet sinon de remplacer avantageusement les étapes de lavages à l'eau classiquement mises en oeuvre, en tout cas d'en limiter le nombre d'étapes nécessaires pour obtenir une bonne séparation.

De plus, le rendement de récupération de l'acide nitrique est très bon puisqu'il est possible de récupérer au moins 95 %, voire la totalité de l'acide nitrique contenu dans le flux de composé dinitré aromatique.

Ce résultat a une conséquence directe sur la qualité des effluents aqueux provenant des étapes de lavages subséquentes des composés nitrés aromatiques, qui comprennent encore l'acide sulfurique et des sous-produits organiques. En effet, ces effluents présentent des teneurs en nitrates considérablement diminuées, ce qui simplifie les étapes de traitement des eaux résiduaires avant leur rejet.

Enfin, le procédé selon l'invention permet de récupérer tes vapeurs nitreuses.

Mais d'autres avantages et caractéristiques de la présente invention apparaîtront plus clairement à la lecture de la description qui va suivre.

Ainsi que cela a été indiqué auparavant, la présente invention a pour objet la séparation et la récupération d'acide nitrique contenu dans un flux à base d'un composé aromatique nitré.

Par composé aromatique nitré, on désigne un composé obtenu par dinitration d'un composé aromatique. Il est à noter que dans ce qui va suivre, il sera fait indifféremment référence à des composés dinitrés ou à des composés nitrés.

Les composés aromatiques susceptibles d'être nitrés peuvent comprendre un ou plusieurs noyaux aromatiques. Conviennent par exemple le benzène et ses dérivés, le naphtalène et ses dérivés, le phénanthrène et ses dérivés, le biphényle, l'oxyde de diphényle et leurs dérivés.

Par dérivés, on désigne des radicaux aromatiques comportant un ou plusieurs substituants, tels que les radicaux alkyle en C₁-C₆ ou cycloalkyle en C₃-C₆ ; des radicaux hydroxyle ; des radicaux alcoxy en C₁-C₅ ; des radicaux aminoacylés en C₁-C₄; des atomes d'halogène.

A titre d'exemple de radical alkyle ou cycloalkyle, on peut citer, sans intention de s'y limiter, le méthyle, l'éthyle, le n-propyle, l'isopropyle, le n-butyle, l'isobutyle, le tertiobutyle, le n-hexyle, le cyclohexyle.

En ce qui concerne les radicaux alcoxy, on peut citer par exemple, le méthoxy, l'éthoxy, le propoxy.

En tant que radicaux aminoacylés, conviennent notamment les radicaux acétylamine, benzoylamine.

Tous les halogènes conviennent, que ce soit le fluor, le chlore, le brome et l'iode.

De préférence, le composé aromatique mis en oeuvre dans les réactions de nitration, est choisi parmi le benzène, le toluène, le xylène et ses isomères, l'éthylbenzène, le propylbenzène, l'isopropylbenzène, le chlorobenzène, le chlorométhylbenzène et ses isomères, le chloréthylbenzène et ses isomères, le biphényle, l'oxyde de diphényle.

Selon un mode de réalisation particulier, le flux traité conformément à la présente invention, est issu de la dinitration du benzène, ou de préférence du toluène.

Le flux comprenant le composé aromatique dinitré est donc obtenu en effectuant une réaction de nitration du composé aromatique correspondant, en présence d'un acide nitrant sulfonitrique. Ainsi, l'acide nitrant comprend plus particulièrement un mélange d'acide nitrique et d'acide sulfurique. Puis les composés aromatiques dinitrés sont ensuite séparés de l'acide nitrant résiduel, pour donner le flux qui sera traité conformément au procédé selon l'invention.

Avantageusement, cette opération a lieu par simple décantation ou centrifugation du mélange réactionnel.

Habituellement, les composés aromatiques dinitrés ainsi débarrassés de la majeure partie de l'acide nitrant, sont qualifiés de composés dinitrés aromatiques bruts.

Selon la première étape du procédé selon l'invention, on effectue une distillation ou un stripping, du flux comprenant le composé aromatique dinitré.

Ainsi que cela a été indiqué auparavant, le flux traité correspond au composés dinitrés bruts.

Plus particulièrement, le flux traité conformément à l'invention présente une teneur en composés aromatiques dinitrés au moins égale à 80 % par rapport au poids total du flux, et de façon plus avantageuse, au moins égale à 90 % par rapport à la même référence.

A titre d'indication, ledit flux présente une teneur en acide nitrique inférieure à 10 % en poids par rapport au poids total du flux et des vapeurs nitreuses. De préférence, la teneur en acide nitrique dans le flux à traiter est inférieure à 5 % en poids par rapport à la même référence.

Le flux traité peut comprendre en outre de l'acide sulfurique.

Selon une première variante, la teneur en acide sulfurique est inférieure à 3 % en poids par rapport aux composés dinitrés aromatiques. Cette teneur en acide sulfurique est classique pour des mélanges réactionnels issus d'une nitration en mélange sulfonitrique.

Selon une seconde variante, la teneur en acide sulfurique est telle qu'après l'extraction de l'acide nitrique, réalisée conformément à l'étape (a) du procédé de l'invention, le flux comprenant les composés nitrés décante. Généralement, ladite teneur est supérieure à 3 % en poids par rapport aux composés dinitrés. Dans le cadre de cette variante, il est nettement avantageux de recycler l'acide sulfurique mis en oeuvre. Par exemple, il peut être joint au flux d'acide résiduaire du procédé de nitration pour être éventuellement concentré. Par la suite, ce flux est alimenté à nouveau dans le processus de nitration.

Enfin, le flux à traiter peut comprendre de l'eau. Cette teneur est variable et dépend de la teneur en acide sulfurique. A titre indicatif, et dans le cas où la teneur en acide sulfurique est faible, la teneur en eau est inférieure à 0,3 % par rapport aux composés dinitrés aromatiques.

Habituellement, l'opération est effectuée de manière à séparer la quantité présente d'acide nitrique. Généralement elle est équivalente au plus à 10% en poids par rapport au poids de composés dinitrés aromatiques. De préférence, cette quantité correspond à 1 à 5 % en poids par rapport à la même référence.

L'étape (a) est mise en oeuvre plus particulièrement dans une colonne comprenant 1 à 4 plateaux théoriques.

De manière tout à fait avantageuse, l'étape (a) a lieu dans une colonne ne comprenant qu'un étage théorique (flash).

Selon un second mode de réalisation, l'étape (a) est effectuée dans un évaporateur à film.

L'étape (a) peut en outre avoir lieu en présence ou en l'absence d'agitation. De préférence, l'opération de séparation est mise en oeuvre en l'absence d'agitation.

Une variante particulière de la présente invention consiste à mettre en oeuvre l'étape (a) de séparation sous une pression comprise entre 10² Pa et la pression atmosphérique (voisine de 10⁵ Pa).

Selon un premier mode de réalisation de cette variante, on effectue une distillation sous vide. Plus particulièrement, la séparation est effectuée sous une pression comprise entre 10³ et 10⁴ Pa.

Selon un second mode de réalisation de cette variante, on effectue un stripping, c'est-à-dire une séparation par entraînement de l'acide nitrique avec un gaz vecteur.

Selon ce mode de réalisation, la pression est voisine de la pression atmosphérique.

En outre, le gaz vecteur est de manière avantageuse et préférée, l'azote. Les gaz rares comme l'hélium, l'argon peuvent de même convenir à la réalisation de cette étape. De préférence, le gaz vecteur est sec.

Il est à noter que même dans le cadre d'une distillation sous vide, il est préférable de maintenir une atmosphère contrôlée, sèche et inerte dans l'appareillage.

L'étape (a) est mise en oeuvre à une température supérieure à la température de fusion des composés nitrés présents dans le flux. Par ailleurs, on se place à une température inférieure à celle de dégradation des composants du flux traité, c'est-à-dire essentiellement des composés nitrés aromatiques. Bien évidemment, cette température est non seulement fonction des conditions opératoires de la séparation, des composés nitrés aromatiques mais est aussi fonction du fait que l'on souhaite séparer l'acide nitrique sans entraîner les composés nitrés aromatiques.

Dans le cadre particulier du dinitrotoluène et ses isomères, cette température est inférieure à 130°C. Si l'on opère la séparation dans les conditions de pression réduite indiquées ci-dessus, la température à laquelle est conduite la distillation de l'étape (a) est comprise entre 50 et 100°C. Plus particulièrement, la température est comprise entre 60 et 80°C.

Le flux récupéré en tête de distillation ou de stripping, et comprenant l'acide nitrique, peut être avantageusement recyclé.

Ainsi, une première variante de l'étape (b) du procédé conforme à l'invention, consiste à recycler ledit flux récupéré en tête de la distillation ou de stripping, et comprenant l'acide nitrique, dans le procédé de nitration.

Par exemple, le flux en tête de distillation ou de stripping peut être recyclé directement en tout endroit du procédé de nitration.

Il est à noter que s'il s'agit d'une dinitration, conduite en deux étapes, le flux peut être indifféremment recyclé à la réaction de mononitration, ou bien à la réaction de dinitration. De préférence, le flux en question est renvoyé à la réaction de mononitration.

Il peut de même être absorbé, sans condensation préalable, dans de l'acide sulfurique et/ou de l'eau avant d'être recyclé dans le procédé de nitration.

Il est de même possible de traiter ce flux de manière à récupérer les vapeurs nitreuses, en mettant en oeuvre tout procédé classique de récupération des vapeurs nitreuses, comme les procédés d'oxydo-absorption sous pression par exemple.

Enfin, l'étape (c) du procédé selon l'invention consiste à traiter le flux récupéré en pied de la distillation ou du stripping, et qui comprend les composés dinitrés aromatiques, l'acide sulfurique et les sous-produits organiques, de manière à séparer les premiers de l'acide sulfurique et des sous-produits.

Cette opération a lieu de manière classique en mettant en oeuvre un ou plusieurs lavages.

Dans le cas où la quantité d'acide sulfurique présente dans le flux issu de l'étape (a) est supérieure à 3% par rapport aux composés dinitrés aromatiques, on effectue l'étape (c) en séparant au préalable l'acide sulfurique présent dans ce flux.

Ceci peut être mis en oeuvre selon toute méthode connu de l'homme de l'art, comme la décantation, la centrifugation.

De manière avantageuse, l'acide sulfurique ainsi séparé peut être recyclé dans le procédé de nitration, comme indiqué auparavant.

L'étape (c) va maintenant être décrite.

Ainsi, selon un mode de réalisation particulier qui ne peut être considéré comme une limitation à l'invention, on effectue un premier lavage à l'eau de manière à éliminer la majeure partie de l'acide sulfurique présent. L'acide sulfurique résultant (acide sulfurique dilué) pourra être avantageusement recyclé dans le procédé. Par exemple, il peut être réintroduit dans le procédé de nitration proprement dit, de préférence après avoir subi une étape de concentration, seul ou en combinaison avec un autre flux, comme notamment l'acide résiduaire de mononitration. Il peut de même être recyclé dans l'étape de lavage elle-même (boucle). La combinaison de ces deux variantes est possible.

Si cette étape de lavage est optionnelle, elle reste néanmoins conseillée pour une bonne rentabilité du procédé.

Dans une seconde étape, on lave le flux de composés nitrés en présence d'une solution aqueuse d'un agent alcalin (lavage basique). Une telle opération a pour but de transformer l'acide sulfurique et les sous-produits, qui sont principalement des composés hydroxynitroaromatiques, en sels solubles dans la phase aqueuse.

Cet agent alcalin peut être choisi parmi les hydroxydes, le carbonates de métaux alcalins ou alcalino-terreux.

Que ce soit pour le premier lavage optionnel à l'eau, ou pour le second en présence d'un agent alcalin, ces opérations peuvent avoir en une ou plusieurs étapes, à contre-courant de préférence, si l'option mettant en oeuvre des étapes multiples est choisie.

Il suffit ensuite, quel que soit le lavage mis en oeuvre, de séparer la phase organique comprenant les composé nitrés, de la phase aqueuse.

En ce qui concerne ce procédé de lavage basique, on pourra se référer à la demande de brevet européen EP 662 454.

Le mode de réalisation détaillé dans la demande de brevet ci-dessus mentionnée, est particulièrement avantageux en ce sens que les eaux basiques chargées en sels polluants peuvent être détruites directement après leur sortie du circuit de lavage, cela sans occasionner de coûts élevés.

Il est cependant possible de traiter ces effluents aqueux avec des agents oxydants détruisant les sous-produits organiques, avant de rejeter les eaux. Parmi les agents oxydants appropriés, on peut citer l'hypochlorite de sodium, l'eau oxygénée. L'emploi de tels agents a notamment été décrit dans la demande de brevet européen EP 654 463. Mais d'autres voies de traitements sont également envisageables.

Les composés nitrés aromatiques ayant subi l'étape de lavage basique, peuvent ensuite être lavés à nouveau, mais avec de l'eau, de manière à ôter toute trace d'agent alcalin.

Là encore, l'opération peut avoir lieu en une ou plusieurs étapes, à contre-courant de préférence, s'il s'agit d'un lavage en plusieurs étapes.

A l'issue de ces diverses étapes de lavages, les composés nitrés aromatiques, et plus particulièrement les composés issus de la dinitration du toluène, peuvent être convenablement utilisés pour préparer les amines correspondantes, par hydrogénation en présence d'un catalyseur à base de nickel, notamment.

Un exemple concret mais non limitatif de l'invention va maintenant être donné.

### EXEMPLE

On alimente un évaporateur à film LUWA (surface 1,6 dm²), avec un débit de 550 ml/h de dinitrotoluène brut de composition suivante :
* 2,35 % d'acide nitrique (poids / poids de dinitrotoluène),
* 0,3 % d'acide sulfurique (poids / poids de dinitrotoluène),
* traces d'eau
* vapeurs nitreuses
* qsp 100% de dinitrotoluène.

La séparation est effectuée sous une pression de 2.10³ Pa (20 mbar), la température de la double enveloppe est maintenue à 65°C.

En deux passages (équivalent à un passage unique sur une surface d'évaporateur de 3,2 dm²), on récupère 97 % de l'acide nitrique et tout le NO₂ dissous.

Les analyses montrent qu'il n'y a pas de dégradation du dinitrotoluène en trinitrotoluène.

## Revendications

1. Procédé de traitement d'un flux obtenu par (1) réaction de dinitration de composés aromatiques avec un acide nitrant comprenant au moins de l'acide nitrique, puis (2) séparation de l'acide nitrant ; ce flux comprenant un composé dinitré aromatique dans lequel reste dissous une fraction d'acide nitrant ; procédé comprenant la mise en oeuvre des étapes suivantes :
(a) On effectue une distillation ou un stripping du flux précité,
(b) On recycle l'acide nitrique récupéré en tête de la distillation ou du stripping, dans le procédé de nitration,
(c) On traite le flux débarrassé de l'acide nitrique et comprenant entre autres les composés aromatiques dinitrés par lavage.

2. Procédé selon la revendication précédente, **caractérisé en ce que** l'on met en oeuvre la distillation de l'étape (a) sur un flux dont la teneur en composés aromatiques dinitrés est au moins égale à 80 % par rapport au poids total du flux.

3. Procédé selon l'une quelconque des revendications précédentes, **caractérisé en ce que** l'on effectue l'étape (a) sous une pression comprise entre 10² Pa et la pression atmosphérique.

4. Procédé selon l'une quelconque des revendications précédentes, **caractérisé en ce que** l'on effectue une distillation sous une pression comprise entre 10³ et 10⁴ Pa.

5. Procédé selon l'une quelconque des revendications 1 ou 3, **caractérisé en ce que** l'on effectue un stripping à une pression voisine de la pression atmosphérique, en présence d'un gaz vecteur.

6. Procédé selon la revendication précédente, **caractérisé en ce que** l'on met en oeuvre le stripping en présence d'un gaz vecteur inerte, comme l'azote ou un gaz rare, comme l'hélium ou l'argon.

7. Procédé selon l'une quelconque des revendications précédentes, **caractérisé en ce que** l'on met en oeuvre la distillation de l'étape (a) à une température supérieure à la température de fusion des composés aromatiques dinitrés présents dans le flux.

8. Procédé selon l'une quelconque des revendications précédentes, **caractérisé en ce que** l'on met en oeuvre l'étape (a) dans une colonne comprenant 1 à 4 plateaux théoriques.

9. Procédé de traitement selon l'une quelconque des revendications 1 à 7, **caractérisé en ce que** l'on effectue l'étape (a) dans un évaporateur à film.

10. Procédé de traitement selon l'une quelconque des revendications précédentes, **caractérisé en ce que** l'on effectue l'étape (a) avec un flux comprenant de l'acide sulfurique, avec une teneur inférieure à 3 % en poids par rapport aux composés dinitrés aromatiques.

11. Procédé de traitement selon l'une quelconque des revendications 1 à 9, **caractérisé en ce que** l'on effectue l'étape (a) avec un flux comprenant de l'acide sulfurique, avec une teneur supérieure à 3% en poids par rapport aux composés dinitrés aromatiques.

12. Procédé selon l'une quelconque des revendications précédentes, **caractérisé en ce que** l'on recycle le flux récupéré en tête de distillation ou de stripping directement en tout endroit du procédé de nitration.

13. Procédé selon l'une quelconque des revendications précédentes, **caractérisé en ce que** l'on recycle dans le procédé de nitration, le flux récupéré en tête de distillation ou de stripping, après l'avoir absorbé, avec ou sans condensation préalable, dans de l'acide sulfurique et/ou de l'eau.

14. Procédé selon l'une quelconque des revendications précédentes, **caractérisé en ce que** l'on traite le flux récupéré en tête de distillation ou de stripping, éventuellement après l'avoir condensé et/ou absorbé dans l'acide sulfurique, l'eau ou leurs mélange, dans tout procédé de récupération des vapeurs nitreuses.

15. Procédé selon l'une quelconque des revendications 1 à 9, et 11 à 14, **caractérisé en ce que**, si la teneur en acide sulfurique est supérieure à 3 % en poids par rapport aux composés dinitrés aromatiques, dans le flux issu de l'étape (a), on effectue une étape préalable de séparation de l'acide sulfurique avant l'étape (c).

16. Procédé selon l'une quelconque des revendications 1 à 9, et 11 à 15, **caractérisé en ce que** l'on effectue l'étape (c) en séparant au préalable le flux issu de l'étape (a), de l'acide sulfurique, ce dernier étant concentré avant d'être alimenté à nouveau dans la processus de nitration.

17. Procédé selon l'une quelconque des revendications précédentes, **caractérisé en ce que** l'on traite un flux issu de la réaction de dinitration de composés aromatiques comprenant un ou plusieurs noyaux aromatiques, et éventuellement comportant un ou plusieurs substituants, tels que les radicaux alkyle C₁-C₆ ou cycloalkyle en C₃-C₆ ; des radicaux hydroxyle ; des radicaux alcoxy en C₁-C₅ ; des radicaux aminoacylés en C₁-C₄; des atomes d'halogène.

18. Procédé selon l'une quelconque des revendications précédentes, **caractérisé en ce que** l'on traite un flux issu de la réaction de dinitration du benzène, du toluène.

## Patentansprüche

1. Verfahren zur Behandlung eines Stroms, erhalten durch (1) eine Dinitrierungsreaktion von aromatischen Verbindungen mit einer nitrierenden Säure, die mindestens Salpetersäure umfasst, dann (2) Abtrennen der nitrierenden Säure; wobei dieser Strom eine dinitrierte aromatische Verbindung enthält, in der ein Anteil der nitrierenden Säure gelöst bleibt; wobei das Verfahren das Ausführen der folgenden Schritte umfasst:
(a) man führt eine Destillation oder ein Abstrippen des vorerwähnten Stroms aus,
(b) man führt die am Kopf der Destillation oder des Abstrippens rückgewonnene Salpetersäure in das Nitrierungsverfahren zurück,
(c) man behandelt den von Salpetersäure befreiten Strom, der unter anderem die dinitrierten aromatischen Verbindungen enthält, durch Waschen.

2. Verfahren nach dem vorangegangenen Anspruch, **dadurch gekennzeichnet, dass** man die Destillation des Schritts (a) an einem Strom, dessen Gehalt an dinitrierten aromatischen Verbindungen bezogen auf das Gesamtgewicht des Stroms mindestens 80% beträgt, ausführt.

3. Verfahren nach einem der vorangegangenen Ansprüche, **dadurch gekennzeichnet, dass** man den Schritt (a) unter einem Druck zwischen 10² Pa und Atmosphärendruck ausführt.

4. Verfahren nach einem der vorangegangenen Ansprüche, **dadurch gekennzeichnet, dass** man eine Destillation unter einem Druck zwischen 10³ und 10⁴ Pa ausführt.

5. Verfahren nach einem der Ansprüche 1 bis 3, **dadurch gekennzeichnet, dass** man ein Abstrippen bei einem Druck nahe dem Atmosphärendruck in Gegenwart eines Trägergases ausführt.

6. Verfahren nach dem vorangegangenen Anspruch, **dadurch gekennzeichnet, dass** man das Abstrippen in Gegenwart eines inerten Trägergases, wie Stickstoff oder ein Edelgas, wie Helium oder Argon, ausführt.

7. Verfahren nach einem der vorangegangenen Ansprüche, **dadurch gekennzeichnet, dass** man die Destillation des Schritts (a) bei einer Temperatur über der Schmelztemperatur der dinitrierten aromatischen Verbindungen, die in dem Strom vorhanden sind, ausführt.

8. Verfahren nach einem der vorangegangenen Ansprüche, **dadurch gekennzeichnet, dass** man den Schritt (a) in einer Kolonne, die 1 bis 4 theoretische Böden enthält, ausführt.

9. Behandlungsverfahren nach einem der Ansprüche 1 bis 7, **dadurch gekennzeichnet, dass** man den Schritt (a) in einem Dünnschichtverdampfer ausführt.

10. Behandlungsverfahren nach einem der vorangegangenen Ansprüche, **dadurch gekennzeichnet, dass** man den Schritt (a) mit einem Strom, der Schwefelsäure in einem Gehalt unter 3 Gew.-% bezogen auf die dinitrierten aromatischen Verbindungen enthält, ausführt.

11. Behandlungsverfahren nach einem der Ansprüche 1 bis 9, **dadurch gekennzeichnet, dass** man den Schritt (a) mit einem Strom, der Schwefelsäure in einem Gehalt über 3 Gew.-% bezogen auf die dinitrierten aromatischen Verbindungen enthält, ausführt.

12. Verfahren nach einem der vorangegangenen Ansprüche, **dadurch gekennzeichnet, dass** man den am Kopf der Destillation oder des Abstrippens rückgewonnenen Strom direkt in den Ort des Nitrierungsverfahrens zurückführt.

13. Verfahren nach einem der vorangegangenen Ansprüche, **dadurch gekennzeichnet, dass** man den am Kopf der Destillation oder des Abstrippens rückgewonnenen Strom in das Nitrierungsverfahren zurückführt, nachdem man ihn, mit oder ohne zuvor erfolgte Kondensation, in Schwefelsäure und/oder Wasser absorbiert hat.

14. Verfahren nach einem der vorangegangenen Ansprüche, **dadurch gekennzeichnet, dass** man den am Kopf der Destillation oder des Abstrippens rückgewonnenen Strom, gegebenenfalls nachdem man ihn kondensiert und/oder in Schwefelsäure, Wasser oder deren Mischungen absorbiert hat, in einem jeglichen Verfahren zur Gewinnung der nitrosen Gase behandelt.

15. Verfahren nach einem der Ansprüche 1 bis 9 und 11 bis 14,
**dadurch gekennzeichnet, dass**, wenn der Gehalt an Schwefelsäure über 3 Gew.-% bezogen auf die dinitrierten aromatischen Verbindungen beträgt, man an dem aus Schritt (a) hervorgehenden Strom einen vorab erfolgenden Schritt zur Abtrennung der Schwefelsäure vor Schritt (c) ausführt.

16. Verfahren nach einem der Ansprüche 1 bis 9 und 11 bis 15,
**dadurch gekennzeichnet, dass** man den Schritt (c) ausführt, indem man zuerst den aus Schritt (a) hervorgehenden Strom von Schwefelsäure abtrennt, wobei diese letztere aufkonzentriert wird, bevor sie erneut in den Nitrierungsprozess eingespeist wird.

17. Verfahren nach einem der vorangegangenen Ansprüche, **dadurch gekennzeichnet, dass** man einen Strom behandelt, der aus der Dinitrierungsreaktion von aromatischen Verbindungen stammt, die einen oder mehrere aromatische Ringe enthalten und gegebenenfalls einen oder mehrere Substituenten, wie C₁-C₆-Alkyl- oder C₃-C₆-Cycloalkylreste, Hydroxylreste, C₁-C₅-Alkoxyreste, C₁-C₄-Aminoacylreste, Halogenatome, enthalten.

18. Verfahren nach einem der vorangegangenen Ansprüche, **dadurch gekennzeichnet, dass** man eine Strom behandelt, der aus der Dinitrierungsreaktion von Benzol, von Toluol stammt.

## Claims

1. Process for treating a stream obtained by (1) the dinitration reaction of aromatic compounds with a nitrating acid comprising at least nitric acid and then (2) the separation of the nitrating acid, this stream comprising an aromatic dinitrated compound in which a fraction of the nitrating acid remains dissolved, the process comprising the implementation of the following steps:
(a) An operation of distilling or stripping the aforementioned stream is carried out;
(b) The nitric acid recovered at the top of the distillation or stripping plant is recycled into the nitration process; and
(c) The stream freed of the nitric acid and comprising, inter alia, the dinitrated aromatic compounds, is treated by washing.

2. Process according to the preceding claim, **characterized in that** the distillation in step (a) is carried out on a stream whose content of dinitrated aromatic compounds is at least equal to 80% with respect to the total weight of the stream.

3. Process according to any one of the preceding claims, **characterized in that** step (a) is carried out under a pressure of between 10² Pa and atmospheric pressure.

4. Process according to any one of the preceding claims, **characterized in that** a distillation operation is carried out under a pressure of between 10³ and 10⁴ Pa.

5. Process according to either of claims 1 and 3, **characterized in that** a stripping operation is carried out at a pressure close to atmospheric pressure, in the presence of a carrier gas.

6. Process according to the preceding claim, **characterized in that** the stripping operation is carried out in the presence of an inert carrier gas, such as nitrogen, or a rare gas, such as helium or argon.

7. Process according to any one of the preceding claims, **characterized in that** the distillation in step (a) is carried out at a temperature greater than the melting point of the dinitrated aromatic compounds present in the stream.

8. Process according to any one of the preceding claims, **characterized in that** step (a) is carried out in a column comprising 1 to 4 theoretical plates.

9. Treatment process according to any one of claims 1 to 7, **characterized in that** step (a) is carried out in a film evaporator.

10. Treatment process according to any one of the preceding claims, **characterized in that** step (a) is carried out with a stream comprising sulphuric acid, with a content of less than 3% by weight with respect to the aromatic dinitrated compounds.

11. Treatment process according to any one of claims 1 to 9, **characterized in that** step (a) is carried out with a stream comprising sulphuric acid, with a content greater than 3% by weight with respect to the aromatic dinitrated compounds.

12. Process according to any one of the preceding claims, **characterized in that** the stream recovered at the top of the distillation or stripping plant is recycled directly into any point in the nitration process.

13. Process according to any one of the preceding claims, **characterized in that** the stream recovered at the top of the distillation or stripping plant is recycled into the nitration process after it has been absorbed, with or without prior condensation, in sulphuric acid and/or water.

14. Process according to any one of the preceding claims, **characterized in that** the stream recovered at the top of the distillation or stripping plant is treated, optionally after it has been condensed and/or absorbed in sulphuric acid, water or a mixture thereof, in any nitrous vapour recovery process.

15. Process according to any one of claims 1 to 9 and 11 to 14, **characterized in that**, if the sulphuric acid content is greater than 3% by weight with respect to the aromatic dinitrated compounds in the stream coming from step (a), a prior step of separating the sulphuric acid is carried out before step (c).

16. Process according to any one of claims 1 to 9 and 11 to 15, **characterized in that** step (c) is carried out after firstly separating the sulphuric acid from the stream coming from step (a), this sulphuric acid being concentrated before being fed again into the nitration process.

17. Process according to any one of the preceding claims, **characterized in that** a stream coming from the dinitration reaction of aromatic compounds comprising one or more aromatic rings, and optionally including one or more substituents such as C₁-C₆ alkyl or C₃-C₆ cycloalkyl radicals, hydroxyl radicals, C₁-C₅ alkoxy radicals, C₁-C₄ aminoacyl radicals, or halogen atoms, is treated.

18. Process according to any one of the preceding claims, **characterized in that** a stream coming from the dinitration reaction of benzene or toluene is treated.
